# EUROPÄISCHE PATENTANMELDUNG

(11) **EP 2 031 083 A1**
(43) Veröffentlichungstag der Anmeldung: **04.03.2009**
(21) Anmeldenummer: 07017236.6
(22) Anmeldetag: 03.09.2007
(51) Int. Cl.: C22C 38/44, C07D 319/12

(54) **Verfahren zur Herstellung von Milchsäuredilactid oder daraus herstellbaren Polyestern**

(71) Anmelder: Uhde Inventa-Fischer GmbH, 13509 Berlin (DE)
(72) Erfinder: Hagen, Rainer, Dr., 13465 Berlin (DE); Mühlbauer, Udo, 10119 Berlin (DE)
(74) Vertreter: Pfenning, Meinig & Partner GbR

(57) **Zusammenfassung**

Vorliegende Erfindung betrifft ein Verfahren zur Herstellung oder Reinigung von bioabbaubaren, intramolekularen cyclischen Estern von alpha-Hydroxycarbonsäuren, bioabbaubaren alpha-Hydroxycarbonsäuren bzw. Oligo- oder Polymeren davon, sowie aus den vorgenannten Verbindungen herstellbaren bioabbaubaren Polyestern.

## Beschreibung

Vorliegende Erfindung betrifft ein Verfahren zur Herstellung oder Reinigung von bioabbaubaren, intramolekularen cyclischen Estern von alpha-Hydroxycarbonsäuren, bioabbaubaren alpha-Hydroxycarbonsäuren bzw. Oligo- oder Polymeren davon, sowie aus den vorgenannten Verbindungen herstellbaren bioabbaubaren Polyestern.

Angesichts stetig steigender Rohstoffpreise sowie der Verknappung von Erdölressourcen stellen Polylactide als bioabbaubare und somit umweltneutrale Kunststoffe eine stetig attraktiver werdende Alternative zu herkömmlichen Kunststoffen dar. Durch mittlerweile vielfältige Einstellungsmöglichkeiten des Molekulargewichts bzw. der optischen Reinheit von Polylactiden sind vielseitige Anwendungsmöglichkeiten zugänglich geworden, beispielsweise in der Verpackungsindustrie oder für medizinische Anwendungen.

Somit besteht ein stetig steigendes Interesse an Verfahren wie der Herstellung oder der Reinigung von Edukten wie beispielsweise Milchsäure, Lactoylmilchsäure oder Dilactid zur Herstellung von Polylactiden. Da während der gesamten Verfahrenskette der Polylactidherstellung immer Milchsäure vorhanden ist, geht dies mit gesteigerten Anforderungen an die Materialien der Reaktoren einher, da Milchsäure eine mittelstarke Säure mit einem pKs-Wert von 3,86 ist und somit, vor allem bei gesteigerten Temperaturen, wie sie normalerweise bei der Prozessführung auftreten, zur Korrosion von normalen Werkstoffen, wie beispielsweise Eisen, befähigt ist.

Bei den Verfahren aus dem Stand der Technik war es stets nachteilig, dass es bei Verwendung von herkömmlichen Materialien für die Reaktoren zu einer Korrosion der mit der Reaktionsmischung in Kontakt stehenden Bauteile kam. Das führt zum Versagen oder zu kurzer Lebensdauer von Reaktoren, Rohrleitungen, Pumpen, Ventilen oder Armaturen. Dadurch erhöhen sich Anlagenausfallzeiten, Reparaturaufwand, Kontrollaufwand und die Wirtschaftlichkeit des Verfahrens leidet. Außerdem werden die Produkte durch die aufgelösten Metallbestandteile verunreinigt wurden, was zu nachteiligen Produkteigenschaften führt. Durch die enthaltenen Metallsalze kann unter Umständen wieder eine katalytische Zersetzung des gebildeten Produktes einsetzen. Somit ist es Aufgabe vorliegender Erfindung, ein Verfahren bereitszustellen, bei dem die vorgenannten Stoffe in hoher Reinheit erhalten werden können. Beispielsweise sollte das bei einem Polymerisationsprozess entstehende Polylactid für die Verwendung als Spinnpolymer keine Verfärbungen aufweisen, da ansonsten die Herstellung weißer Garne oder Gewebe praktisch unmöglich wird und die gezielte Einstellung von Farben durch Zugabe von Farben oder Pigmenten erschwert wird.

Diese Aufgabe wird gelöst durch die Merkmale des Patentanspruchs 1. Dabei stellen die abhängigen Ansprüche vorteilhafte Weiterbildungen dar. Mit Patentansprüchen 11 und 12 werden Verwendungsmöglichkeiten des Verfahrens genannt.

Erfindungsgemäß wird ein Verfahren zur Herstellung oder Reinigung eines bioabbaubaren, intermolekularen cyclischen Diesters einer alpha-Hydroxycarbonsäure der Formel I, 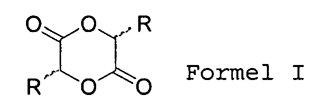 daraus durch Ringöffnungspolymerisation herstellbaren Oligo- oder Polymeren des Diesters der Formel I; einer Säure der allgemeinen Formel II 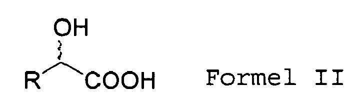 und/oder daraus durch Polykondensation herstellbaren Oligo- oder Polymeren der Säure der Formel II, wobei R ausgewählt ist aus Wasserstoff oder linearen oder verzweigten aliphatischen Kohlenwasserstoffresten mit 1 bis 6 Kohlenstoffatomen, und/oder Mischungen hieraus, in einer Vorrichtung bereitgestellt, wobei zumindest die mit der Reaktionsmischung, die mit den Edukten und/oder die mit den Prozessdämpfen kontaktierten Flächen der Vorrichtung teilweise oder vollständig aus mindestens einer Stahlsorte bestehen, die sowohl eine ferritische als auch eine austenitische Phase enthält, wobei die Stahlsorte
a) 20 bis 30 Gew.-% Chrom
b) 3 bis 10 Gew.-% Nickel
c) 0,1 bis 5 Gew.-% Molybdän
d) 0 bis 0,5 Gew.-% Stickstoff, sowie
e) 0 bis 0,1 Gew.-% Kohlenstoff
   enthält.

Vorteilhafterweise liegt dabei der Chromanteil zwischen 20 und 26 Gew.-%, der Molybdänanteil zwischen 2,0 und 4,5 Gew.-%, der Stickstoffanteil zwischen 0,08 und 0,5 Gew.-%, sowie der Kohlenstoffanteil maximal bei 0,05 Gew.-%.

In einer weiteren vorteilhaften Ausführungsform ist mindestens ein weiteres Element, ausgewählt aus der Gruppe bestehend aus Cu, Mn, Ti, W, Ta Si und/oder Mischungen hieraus zulegiert. Dabei beträgt der Gehalt des mindestens einen Elements insbesondere zwischen 0,1 und 8,0 Gew.-%.

Ganz besonders vorteilhaft ist es, wenn die Stahlsorte ausgewählt ist aus der Gruppe bestehend aus Stahlwerkstoffen mit der Werkstoffnummer 1.4462 (Standard Duplex Stahl X2CrNiMoN22-5-3), 1.4410 (Superduplex Stahl X2CrNiMoN25-7-4) und/oder 1.4593 (ferritisch-austhenitischer Stahl G-X3 CrNiMoCuN24-6).

Das im Voranstehenden beschriebene Verfahren ist mit einer Vielzahl von Vorrichtungen ausführbar, insbesondere einer Vorrichtung, ausgewählt aus der Gruppe bestehend aus Kondensationsvorrichtung, kontinuierlich oder diskontinuierlich betriebenem Rührkessel, Rohrreaktor, Festphasenkondensator, Rektifikationskolonne, Trennwandkolonne, statische Mischelemente und/oder Elemente zur Strömungsbeeinflussung wie Lochscheiben, Verdrängungskörpern und/oder konzentrischen Ringspalten, Pumpen, sowie dazu gehörige Bauteile und Verbindungen, wie Rohrleitungen, Schrauben, Dichtungen, Flansche, Aufsätze, Ventile, Armaturen, Stutzen, Rührer, Fallfilmverdampfer, Dünnschichtverdampfer, Umlaufverdampfer und/oder Kombinationen hieraus.

Bevorzugt ist Diester der Formel I 3,6-Dimethyl-1,4-dioxan-2,5-dion (Dilactid) und die Säure der Formel II Milchsäure.

Erfindungsgemäße Verwendungszwecke findet das Verfahren bei der Herstellung oder Reinigung von bioabbaubaren, intermolekularen cyclischen Diestern der Formel I, bevorzugt Dilactid, besonders bevorzugt *L,L-*Dilactid, und ebenso bei der Herstellung von Polymeren von cyclischen Diestern der Formel I, bevorzugt Polylactid (PLA), besonders bevorzugt *L*-Polylactid (PLLA).

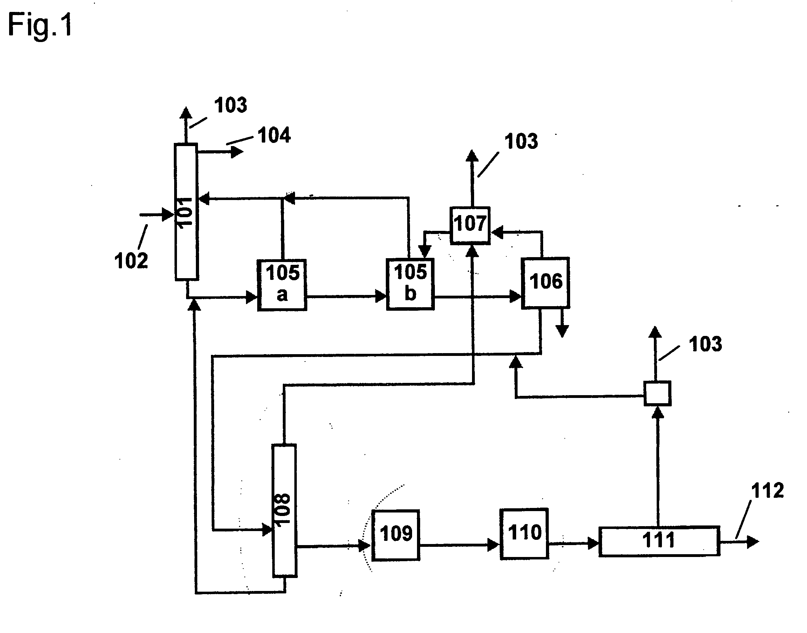

Der Anwendungsbereich der folgenden Erfindung wird anhand der nachfolgenden Fig. 1 näher erläutert, ohne dass die Erfindung auf die in Fig. 1 abgebildeten Prozessschritte und Apparate sowie auf die beschriebene Verfahrensführung beschränkt ist.

Fig. 1 zeigt ein Gesamtschaubild eines Polymerisationsprozesses, bei dem Polylactid erzeugt wird, ausgehend von Milchsäure. Erfindungsgemäß ist dabei vorgesehen, dass zumindest die mit der Reaktionsmischung oder deren Dämpfen in Kontakt stehenden Flächen der im Prozess beteiligten Reaktoren bzw. Vorrichtungen oder auch die verbindenden Leitungen teilweise oder vollständig aus einer im Voranstehenden beschriebenen Stahlsorte bestehen.

Der kontinuierliche Gesamtprozess der Polylactidherstellung (PLA-Prozess) ausgehend von Milchsäure dargestellt untergliedert sich dabei in die folgenden Teilschritte:

### 1. Aufkonzentration von Milchsäure

Das Ausgangsmaterial für den Prozess ist Milchsäure. Dabei muss der Gehalt an Milchsäure höher als 80 Gew.-% sein. Vorzugsweise beträgt dabei die Milchsäurekonzentration mehr als 90 %, weil das Wasser vor der Polymerisation entfernt werden muss. Die Trennung von Wasser und Milchsäure wird dabei in einer Rektifikationssäule 101 vorgenommen. Dabei wird über einen Absaugstutzen 103 Vakuum angelegt, das dampfförmig anfallende Wasser wird kondensiert und über einen weiteren Stutzen 104 kopfseitig entnommen. Die Zuführung der Milchsäure erfolgt dabei kontinuierlich über einen weiteren Stutzen 102. Das Destillat ist reines Wasser, das sumpfseitig anfallende Produkt ist Milchsäure mit einer Konzentration von mehr als 99 Gew.-%.

Neben der Abtrennung von Wasser aus dem Ursprungsmaterial (Milchsäure) dient die Rektifikationssäule 101 ebenso zur Trennung der Dämpfe aus den Präkondensations-Reaktoren 105a und 105b. Die Dampfströme bestehen dabei aus Milchsäure, Lactoylmilchsäure, Dilactid und Wasser. Das Wasser wird kopfseitig abgezogen, Milchsäure und ihre Derivate gehen in den Sumpf der Rektifikationssäule und von dort zusammen mit der aufkonzentrierten Milchsäure in den ersten Präkondensations-Reaktor 105a.

### 2. Präkondensation

Die aufkonzentrierte Milchsäure wird in einer Serie von zwei Reaktoren 105a und 105b durch Polykondensation in ein Präpolymer überführt. Die Polykondensation läuft unter zwei verschiedenen Drücken und Temperaturen ab, um den Reaktionsumsatz zu optimieren. Im ersten Reaktor 105a sind die Konditionen so gewählt, dass die Verdampfung von Milchsäure minimiert ist und gleichzeitig die Entfernung von Wasser erleichtert wird. Im zweiten Schritt der Polykondensation ist die Reaktionsgeschwindigkeit durch eine höhere Temperatur erhöht, gleichzeitig wird der Druck vermindert, um die Wasserkonzentration in der Schmelze weiter zu mindern. Die mittlere Molmasse (Zahlenmittel) des Präpolymers liegt dabei zwischen 500 und 2.000 g/mol.

### 3. Cyclisierende Depolymerisation

Das Präpolymer steht in chemischem Gleichgewicht mit dem cyclischen Dimer der Milchsäure, dem Dilactid. Durch Einstellung von Druck und Temperatur im Depolymerisationsreaktor 106 ist gewährleistet, dass das Lactid kontinuierlich aus dem Präpolymer gebildet wird und verdampft. Der Dampfstrom aus dem Depolymerisationsreaktor 106 besteht hauptsächlich aus Lactid. Wasser, Milchsäure und deren lineare Oligomere sind nur in untergeordneten Mengen vorhanden. Die Dämpfe werden teilweise in der erfindungsgemäßen Kondensationsvorrichtung 107 kondensiert: Wasser und der größte Anteil an Milchsäure bleiben dabei dampfförmig. Das Kondensat enthält zuvorderst das Lactid, Lactoylmilchsäure (das lineare Dimer der Milchsäure) und höhere lineare Oligomere. [Lactid liegt in zwei stereoisomeren Formen vor: das optisch aktive *L,L-*Lactid und das Mesolactid, aus einer Kombination einer *L*(+)- und *D*(-)-Milchsäureeinheit. Die *D*(-)-Einheiten stammen teils aus dem Edukt, teils werden sie durch Racemisierung von *L*(+)-Einheiten während der Präpolymerisation und der Depolymerisation gebildet.]

### 4. Lactid-Reinigung

Während der Ringöffnungspolymerisation hängt das erreichbare Molekulargewicht und somit bedeutende mechanische Eigenschaften des Polylactids vom Reinheitsgrad des Lactids ab. Die Hydroxyl-Gruppen der als Verunreinigung enthaltenen Milchsäure und Lactoylmilchsäure dienen dabei als Ausgangspunkt der Polymerisation. Je höher die Konzentration der Hydroxyl-Gruppen im Lactid ist, desto geringer fällt das erreichbare Molekulargewicht des Polymers aus. Die Konzentration der Hydroxyl-Gruppen im Rohlactid ist nach der cyclisierenden Depolymerisation zu hoch. Das kondensierte Lactid wird in einer Rektifikationssäule 108 bis zur benötigten Hydroxylgruppenkonzentration aufgereinigt. Das gereinigte Lactid wird der Säule 108 als Seitenprodukt entnommen. Das Destillat und das Sumpfprodukt werden dem Prozess an unterschiedlichen Stellen wieder.zugeführt. Neben dem Molekulargewicht des Polylactids werden seine Eigenschaften stark durch den *D*-Gehalt (die Menge an strukturellen Einheiten, die die *D*-Konfiguration aufweisen) beeinflusst.

### 5. Ringöffnungspolymerisation

Die Ringöffnungspolymerisation wird in einem Reaktor unternommen, der aus einer Kombination eines Rührkessels 109 und eines Rohrreaktors 110 gebildet ist. Im ersten Reaktor 109 wird das niedrigviskose Lactid zu PLA polymerisiert mit einer Umsetzungsrate von ca. 50 %. Katalysator und Additive werden homogen in die Schmelze eingemischt.

Im Rohreaktor 110 wird die Polymerisationsreaktion so lange fortgeführt, bis ein chemisches Gleichgewicht zwischen Polymer und Monomer erreicht wird. Die maximale Umsetzung des Monomers beträgt ca. 95 %. Während der Polymerisation erhöht sich die Viskosität auf ca. 10.000 pa·sec.

### 6. Entmonomerisierung

Um ein stabiles Polylactid zu erhalten, ist die Monomerkonzentration von ungefähr 5 Gew.-% in der Schmelze zu hoch. Deswegen muss eine Entmonomerisierung durchgeführt werden. Dies wird durch eine Entgasung der Schmelze in einem Doppelschneckenextruder 111 erreicht. Aufgrund der Tatsache, dass die Ringöffnungspolymerisation eine Gleichgewichtsreaktion ist, wird ein Stabilisator vor der Entmonomerisierung zugegeben, um die Rückbildung des Monomers während und nach der Entgasung zu verhindern.

### 7. Granulierung und Kristallisation

Anschließend an die Entmonomerisierung wird die Schmelze dem Extruder 111 entnommen und in ein Granulat 112 überführt. Dabei können sowohl Stranggranulation oder Unterwasser-Granulation durchgeführt werden. In beiden Fällen muss das PLA-Granulat vor der Trocknung und der Verpackung kristallisiert werden. Die Kristallisation wird bei erhöhten Temperaturen und unter Rühren durchgeführt, solange bis das Granulat nicht mehr aneinander klebt.

## Patentansprüche

1. Verfahren zur Herstellung oder Reinigung eines bioabbaubaren, intermolekularen cyclischen Diesters einer alpha-Hydroxycarbonsäure der Formel I, 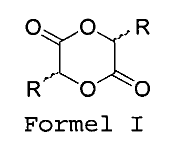 daraus durch Ringöffnungspolymerisation herstellbaren Oligo- oder Polymeren des Diesters der Formel I; einer Säure der allgemeinen Formel II 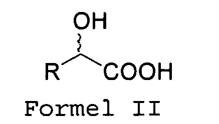 und/oder daraus durch Polykondensation herstellbaren Oligo- oder Polymeren der Säure der Formel II, wobei R ausgewählt ist aus Wasserstoff oder linearen oder verzweigten aliphatischen Kohlenwasserstoffresten mit 1 bis 6 Kohlenstoffatomen, und/oder Mischungen hieraus, in einer Vorrichtung,
**dadurch gekennzeichnet, dass** zumindest die mit der Reaktionsmischung und/oder den Edukten und/oder den Prozessdämpfen kontaktierten Flächen der Vorrichtung teilweise oder vollständig aus mindestens einer Stahlsorte bestehen, die sowohl eine ferritische als auch eine austenitische Phase enthält, wobei die Stahlsorte
a) 20 bis 30 Gew.-% Chrom
b) 3 bis 10 Gew.-% Nickel
c) 0,1 bis 5 Gew.-% Molybdän
d) 0 bis 0,5 Gew.-% Stickstoff, sowie
e) 0 bis 0,1 Gew.-% Kohlenstoff
enthält.

2. Verfahren nach einem der vorhergehenden Ansprüche, **dadurch gekennzeichnet, dass** der Chromanteil zwischen 20 und 26 Gew.-% liegt.

3. Verfahren nach einem der vorhergehenden Ansprüche, **dadurch gekennzeichnet, dass** der Molybdänanteil zwischen 2,0 und 4,5 Gew.-% liegt.

4. Verfahren nach einem der vorhergehenden Ansprüche, **dadurch gekennzeichnet, dass** der Stickstoffanteil zwischen 0,08 und 0,5 Gew.-% liegt.

5. Verfahren nach einem der vorhergehenden Ansprüche, **dadurch gekennzeichnet, dass** der Kohlenstoffanteil maximal 0,05 Gew.-% beträgt.

6. Verfahren nach einem der vorhergehenden Ansprüche, **dadurch gekennzeichnet, dass** mindestens ein weiteres Element, ausgewählt aus der Gruppe bestehend aus Cu, Mn, Ti, W, Ta Si und/oder Mischungen hieraus zulegiert ist.

7. Verfahren nach vorhergehendem Anspruch, **dadurch gekennzeichnet, dass** der Gehalt des mindestens einen Elements zwischen 0,1 und 8,0 Gew.-% beträgt.

8. Verfahren nach einem der vorhergehenden Ansprüche, **dadurch gekennzeichnet, dass** die Stahlsorte ausgewählt ist aus der Gruppe bestehend aus Stahlwerkstoffen mit der Werkstoffnummer 1.4462 (Standard Duplex Stahl X2CrNiMoN22-5-3), 1.4410 (Superduplex Stahl X2CrNiMoN25-7-4) und/oder 1.4593 (ferritisch-austhenitischer Stahl G-X3 CrNiMoCuN24-6).

9. Verfahren nach einem der vorhergehenden Ansprüche, **dadurch gekennzeichnet, dass** die Vorrichtung ausgewählt ist aus der Gruppe bestehend aus Kondensationsvorrichtung, kontinuierlich oder diskontinuierlich betriebenem Rührkessel, Rohrreaktor, Festphasenkondensator, Rektifikationskolonne, Trennwandkolonne, statische Mischelemente und/oder Elemente zur Strömungsbeeinflussung wie Lochscheiben, Verdrängungskörpern und/oder konzentrischen Ringspalten, Pumpen, sowie dazu gehörige Bauteile und Verbindungen, wie Rohrleitungen, Schrauben, Dichtungen, Flansche, Ventile, Armaturen, Aufsätze, Stutzen, Rührer, Fallfilmverdampfer, Dünnschichtverdampfer, Umlaufverdampfer und/oder Kombinationen hieraus.

10. Verfahren nach einem der vorhergehenden Ansprüche, **dadurch gekennzeichnet, dass** der Diester der Formel I 3,6-Dimethyl-1,4-dioxan-2,5-dion (Dilactid) und die Säure der Formel II Milchsäure ist.

11. Verwendung eines Verfahrens nach einem der vorhergehenden Ansprüche bei der Herstellung oder Reinigung von bioabbaubaren, intermolekularen cyclischen Diestern der Formel I, bevorzugt Dilactid, besonders bevorzugt L,L-Dilactid.

12. Verwendung des Verfahren nach einem der Ansprüche 1 bis 10 bei der Herstellung von Polymeren von cyclischen Diestern der Formel I, bevorzugt Polylactid (PLA), besonders bevorzugt L-Polylactid (PLLA).
